# EUROPEAN PATENT APPLICATION

(11) **EP 4 732 859 A1**
(43) Date of publication of application: **29.04.2026**
(21) Application number: 23942023.5
(22) Date of filing: 11.07.2023
(51) Int. Cl.: A61M 1/00, A61B 90/00

(54) **WASTE COLLECTION APPARATUS AND WASTE LIQUID COLLECTION AND TREATMENT SYSTEM**

(30) Priority: 20.06.2023 CN 202321593306 U; 20.06.2023 CN 202310740613; 20.06.2023 CN 202321587189 U
(71) Applicant: Amsino Medical (Shanghai) Co., Ltd., Shanghai 201613 (CN)
(72) Inventor: LU, Lei, Shanghai 201613 (CN); LI, Ya, Shanghai 201613 (CN)
(74) Representative: Metida
(86) International application number: PCT/CN2023/106696
(87) International publication number: WO 2024/259755

(57) **Abstract**

A waste collection apparatus (10) and a waste liquid collection and treatment system, the waste collection apparatus (10) comprising a trolley butt-joining portion (16), wherein the trolley butt-joining portion (16) forms an adapting space (161), and the trolley butt-joining portion (16) comprises a trolley-joint fixing plate (163), a collection end joint (165), a suspension joint (167) and a suspension-joint fixing member (169); the suspension-joint fixing member (169) is fixed to the trolley-joint fixing plate (163); the collection end joint (165) is connected to the suspension joint (167); the suspension joint (167) is mounted on the suspension-joint fixing member (169) in a suspended and movable manner, and the suspension joint (167) is configured to guide a butt-joint end joint of a butt-joining apparatus (30) to engage with the collection end joint (165); and the suspension-joint fixing member (169) comprises a positioning portion (1691), which extends into the adapting space (161). In the waste collection apparatus (10) and the waste liquid collection and treatment system, since the positioning portion (1691) is arranged on the suspension-joint fixing member (169), after the positioning portion (1691) and a guide engagement opening (3471) are positioned, a floating space of the suspension joint (167) has no limiting relationship with an adapter plate (347) and the guide engagement opening (3471), thereby not limiting the floating space of the suspension joint (167), such that a fault-tolerant space during butt joining is large, and the suspension joint (167) cannot be stuck during the butt joining.

## Description

### TECHNICAL FIELD

This invention relates to the technical field of medical equipment, and in particular to a waste collection apparatus and a waste liquid collection and treatment system.

### BACKGROUND

During certain surgical procedures, various types of waste including liquid, semi-solid and solid substances are inevitably generated. Examples include human bodily fluids such as blood, irrigation solutions introduced to the surgical site during the operation, fragments of human tissue, small pieces of surgical materials, and other solid and semi-solid waste produced in the course of surgery. Such waste must be collected in a timely manner to prevent contamination of the surgical site and to avoid it becoming a biological hazard in the operating room or other locations where surgical procedures are performed. In the current state of the art, a vacuum source is typically used to generate suction force, which draws the waste from the surgical site into a designated collection container. Specifically, once the system is activated, the suction force generated by the vacuum source reaches the surgical site, thereby drawing the waste through the tubing connected to the site and into the designated collection container.

In one type of medical waste collection and treatment system, waste materials are collected in a waste collection container connected to a vacuum source. This container is generally mounted on a wheeled portable trolley to facilitate movement and transportation. Generally speaking, during the suction process, when the storage volume of the waste collection container reaches a predetermined level, the container needs to be emptied. In the early state of the art, the practice was to push the waste collection equipment to a docking station for emptying and cleaning. After the waste collection unit is docked to the docking station, the emptying process begins. Once emptying is completed, the waste collection container is cleaned and disinfected by the cleaning system. During a series of medical procedures, emptying is required for each procedure. The frequent operation of pushing the waste collection equipment to the docking station each time causes considerable inconvenience to users and also hinders the progress of medical procedures. Therefore, in order to address this technical drawback, a transfer device specifically designed to transport the waste from the waste collection equipment to the docking station has been developed, namely a docking device dedicated to transferring the waste contained in the waste collection equipment. This device usually includes two connectors that match with the two connectors on the waste collection equipment to establish liquid path communication. One liquid path is used to transfer the waste from the waste collection equipment to the docking device, while the other liquid path is used to clean the waste collection container of the waste collection equipment.

In an example of the prior art, the primary method for the automatic docking of the two connectors on the docking device with the two connectors on the waste collection equipment involves first roughly aligning the positions of the docking device and the waste collection equipment. After the two are roughly connected, the actuator on the docking device moves the two connectors of the docking device in up to six directions (up, down, front, back, left, right) to align the two connectors of the docking device with the two connectors of the waste collection equipment. Since the connectors in the prior art can only move in up, down, front, back, left, right directions at most, the correction can only be performed in four directions (up, down, left, right). After achieving alignment in the four directions (up, down, left, right), the connectors of the docking device can be inserted by moving them forward and backward. In the prior art, the structure for achieving six-directional movement of the connectors on the docking device requires a bracket capable of four-directional movement. This bracket needs to be implemented with multiple elastic elements and various structural parts, plus a stepping motor to achieve movement in the other two directions, resulting in a complex structure and high equipment costs. Further, it requires extremely precise alignment between the docking device and the waste collection equipment before the connectors can be inserted. In other words, the connectors of the docking device and the waste collection equipment must be fully aligned before they come into contact to enable the pluging-in mating operation; alignment cannot be further adjusted after they come into contact. This structure not only complicates the alignment operation but also increases the complexity and cost of the equipment. Meanwhile, due to the low fault tolerance rate, if the alignment is not achieved, the docking of the two connectors of the docking device with the two connectors of the waste collection equipment will cause damage to the connectors.

### SUMMARY

To overcome the drawbacks and deficiencies in the prior art, the object of this invention is to provide a waste collection apparatus and a waste liquid collection and treatment system that can achieve automatic alignment and complete docking during the docking process with a large fault tolerance margin.

The object of this invention is achieved through the following technical solutions:

This invention provides a waste collection apparatus for collecting waste generated during medical procedures and for docking with a docking device, wherein the waste collection apparatus includes a trolley docking portion, the trolley docking portion forms a mating space for accommodating a mating head of the docking device; the trolley docking portion includes a connector mounting plate, a collection-end connector, a floating connector, and a floating connector mounting member, wherein the floating connector mounting member is fixed on the connector mounting plate, the collection-end connector is connected to the floating connector, the floating connector is floatingly movably mounted on the floating connector mounting member, and the floating connector is configured to guide a docking-end connector of the docking device to connect with the collection-end connector, wherein the floating connector mounting member includes a positioning portion extending into the mating space.

In one embodiment, the floating connector is floatingly disposed in a mounting hole formed by the floating connector mounting member, the floating connector includes a floating port and a floating guiding member, the floating port is fixedly connected to the floating guiding member, the floating guiding member is sleeved on the collection-end connector and passes through the floating connector mounting member for guiding the docking-end connector of the docking device; and the floating port is floatingly matched with the mounting hole of the floating connector mounting member to engage with the floating connector mounting member or move relative to the floating connector mounting member.

In one embodiment, a first elastic member is provided between the floating connector mounting member and the floating connector, and the first elastic member is configured to provide an elastic force for resetting the floating connector so as to restore the floating port from a movable state to a state of engaging with the floating connector mounting member.

In one embodiment, the floating connector mounting member includes a fixing top portion, the fixing top portion is fixedly connected to the connector mounting plate, a mating hole is provided in the fixing top portion, at least part of an inner surface of the mating hole faces away from the mating space, and a dimension of the mating hole gradually decreases, the floating port includes a mating surface for mating with the mating hole, the mating surface faces the mating space and is at least partially inclined; and the mating surface of the floating port engages with the inner surface of the mating hole, or disengages from the mating hole as the floating port floats.

In one embodiment, the floating guiding member includes a sleeve portion and a guiding end for guiding the docking-end connector, one end of the sleeve portion is connected to the collection-end connector, the guiding end is connected to the other end of the sleeve portion, the guiding end is provided with a guiding opening, and the guiding opening has a bell-mouth structure.

In one embodiment, a diameter of the guiding opening at its smallest position ranges from 40 mm to 60 mm.

In one embodiment, a diameter of the floating connector ranges from 30 mm to 38 mm, and an inner diameter of the positioning portion ranges from 50 mm to 70 mm.

In one embodiment, one end of the floating guiding member protrudes from one end of the floating connector mounting member into the mating space, the floating guiding member further includes a limiting edge that matches with the positioning portion to limit an upward floating range of the floating connector during floating, and the limiting edge is provided on an outer wall of the guiding end.

In one embodiment, a chamfered portion is formed at one end of the guiding end away from the limiting edge.

In one embodiment, the waste collection apparatus further includes a trolley housing, a waste collection container disposed inside the trolley housing, a trolley main frame, and a travel mechanism arranged at the bottom of the trolley main frame, wherein the trolley docking portion is mounted on the trolley main frame.

This invention also provides a waste liquid collection and treatment system including the waste collection apparatus described above and a docking device, wherein the docking device includes a mating head, the mating head is configured to be inserted into the mating space of the trolley docking portion, the mating head includes a docking-end connector for engaging with the collection-end connector and an adapter plate, a guiding groove allowing the docking-end connector to pass through is provided in the adapter plate, and the positioning portion of the floating connector mounting member matches with the guiding groove of the adapter plate.

In one embodiment, the guiding groove is U-shaped, with a wedge-shaped front end, and the guiding groove has an opening width gradually decreasing from the front end to an inner end thereof.

In one embodiment, the mating head further includes an actuator, which is connected to the docking-end connector and configured to drive the docking-end connector to move for docking with the collection-end connector.

In one embodiment, the mating head further includes a moving plate, the docking-end connector is disposed on the moving plate, and the actuator drives the moving plate to move up and down.

In one embodiment, the mating head further includes a bottom plate, support columns, and guiding columns, wherein the moving plate is located between the bottom plate and the adapter plate, the support columns and the guiding columns are fixed relative to the bottom plate, the adapter plate is fixedly connected to the support columns, and the moving plate is movably inserted through the guiding columns.

In one embodiment, a buffer pad is provided at one end of each of the support columns and the guiding columns that is connected to the adapter plate.

In one embodiment, the mating head further includes a first sensor and a second sensor, which are relatively fixed to the adapter plate, the first sensor and the second sensor are arranged at intervals, a sensor stopper is provided on the moving plate, the first sensor and the second sensor are configured to sense the sensor stopper; when the first sensor detects the sensor stopper, the collection-end connector and the docking-end connector have been successfully docked; and when the second sensor detects the sensor stopper, the moving plate and the docking-end connector have returned to their initial positions.

In one embodiment, the mating head further includes a cover plate, the cover plate is movably disposed above the adapter plate, a second elastic member is provided between the adapter plate and the cover plate, and the second elastic member is configured to provide an elastic force for automatically resetting the cover plate to a position covering the adapter plate.

The beneficial effects of this invention are as follows:

In the waste collection apparatus and waste liquid collection and treatment system of this invention, since the positioning portion is provided on the floating connector mounting member, after the positioning portion is aligned with the guiding groove, the floating space of the floating connector is not constrained by the adapter plate and the guiding groove, thereby not limiting the floating space of the floating connector, to ensure a large fault tolerance margin during docking and prevent the floating connector from jamming in the docking process.

### BRIEF DESCRIPTION OF THE DRAWINGS

FIG. 1 is a schematic structural diagram of a waste collection and treatment system according to an embodiment of this invention.
FIG. 2 is a partial structural diagram of the waste collection apparatus in the waste collection and treatment system shown in FIG. 1.
FIG. 3 is a top view of the trolley docking portion of the waste collection apparatus shown in FIG. 2.
FIG. 4 is a cross-sectional view taken along line IV-IV in FIG. 3.
FIG. 5 is an exploded schematic diagram of the trolley docking portion of the waste collection apparatus shown in FIG. 2.
FIG. 6 is a schematic structural diagram of the docking device in the waste collection and treatment system shown in FIG. 1.
FIG. 7 is an exploded schematic diagram of the docking head of the docking device shown in FIG. 6.
FIG. 8 is an assembly schematic diagram of partial components of the docking head of the docking device shown in FIG. 6.
FIG. 9 is a top view of the docking head of the docking device shown in FIG. 6.
FIG. 10 is a cross-sectional view taken along line X-X in FIG. 9.
FIG. 11 is a schematic structural diagram of the waste collection apparatus and the docking device in the waste collection and treatment system shown in FIG. 1 before docking.
FIG. 12 is a schematic diagram of FIG. 11 from another angle.
FIG. 13 is a schematic structural diagram of the waste collection apparatus and the docking device in the waste collection and treatment system shown in FIG. 1 after docking.
FIG. 14 is a front view of FIG. 13.

### DETAILED DESCRIPTION OF THE EMBODIMENTS

To further illustrate the technical means and effects adopted by this invention to achieve the intended purpose, the specific implementation, structure, characteristics and effects of the display panel and waste collection apparatus proposed according to this invention are described in detail below in conjunction with the accompanying drawings and preferred embodiments.

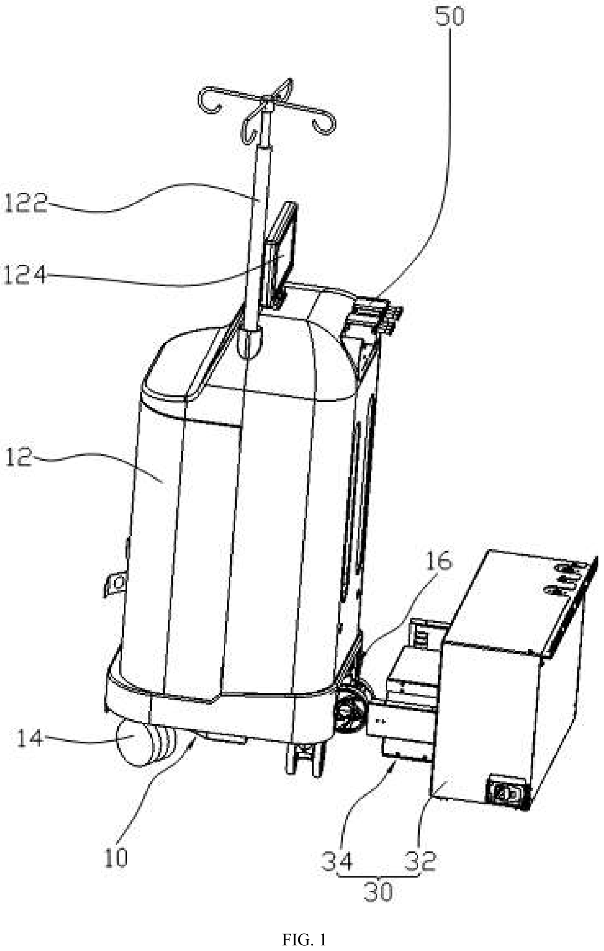

This invention provides a waste collection and treatment system. As shown in FIG. 1, which depicts a waste collection and treatment system according to an embodiment. The waste collection and treatment system in this embodiment includes a waste collection apparatus 10 and a docking device 30, where the docking device 30 is configured to dock with the waste collection apparatus 10. The waste collection apparatus 10 can be connected to an external suction pipeline (not shown) via a consumable box 50, and the external suction pipeline can be connected to a suction connector (not shown). The suction connector may be an independent component or attached to a surgical equipment. The suction force of the suction pipeline may originate from a vacuum source provided inside the waste collection apparatus 10 or an external vacuum source. The suction force at the suction connector delivers waste through the external suction pipeline to be stored in the waste collection apparatus 10. After the docking device 30 is docked with the waste collection apparatus 10, it can empty the waste in the waste collection apparatus 10 and form a cleaning path for cleaning the waste collection container (not shown) of the waste collection apparatus 10. The control of the discharge circuit and flushing circuit of the waste collection and treatment system 10 is known to those skilled in the art and will not be repeated here.

In this embodiment, the waste collection apparatus 10 includes a trolley housing 12, a waste collection container (not shown) disposed inside the trolley housing 12, a trolley main frame 13, a travel mechanism 14 arranged at the bottom of the trolley main frame 13, and a trolley docking portion 16 mounted on the trolley main frame 13. The trolley housing 12 is further provided with a support hanging rod 122 and a collection-end display device 124.

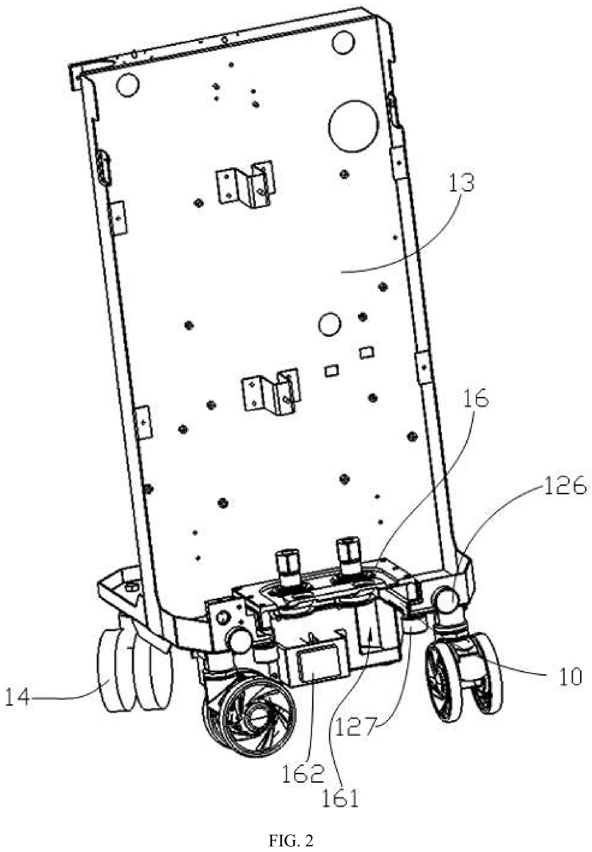

Please also refer to FIG. 2, in this embodiment, the trolley housing 12 of the waste collection apparatus 10 is further provided with a magnetic stopper 126, which is arranged on at least one side of the trolley docking portion 16. Specifically, in this embodiment, one magnetic stopper 126 is provided on each side of the trolley docking portion 16.

Specifically, the trolley housing 12 is further provided with first anti-collision portions 127, which are located on both sides of the trolley docking portion 16. In particular, the first anti-collision portions 127 may be anti-collision rollers.

Specifically, the waste collection container is used to contain the waste sucked by the suction connector.

Specifically, the travel mechanism 14 may include multiple rollers to facilitate the movement of the waste collection apparatus 10, thereby making the waste collection apparatus 10 a portable device for easy use.

In this embodiment, the trolley docking portion 16 may be arranged at the bottom of the trolley main frame 16 and is used for mating with the docking device 30. Specifically, the trolley docking portion 16 forms a mating space 161 to receive the mating head 32 of the docking device 30. The first anti-collision portions 127 are located on both sides of the mating space 161 to prevent the mating head 32 of the docking device 30 from colliding with the inner side walls of the mating space 161.

Specifically, a charging receiving portion 162 is further provided in the mating space 161. The charging receiving portion 162 may be a wireless charging receiving portion 162 to realize wireless charging; alternatively, it may also be a wired plug-in portion, as long as charging can be achieved simultaneously when docked with the docking device 30.

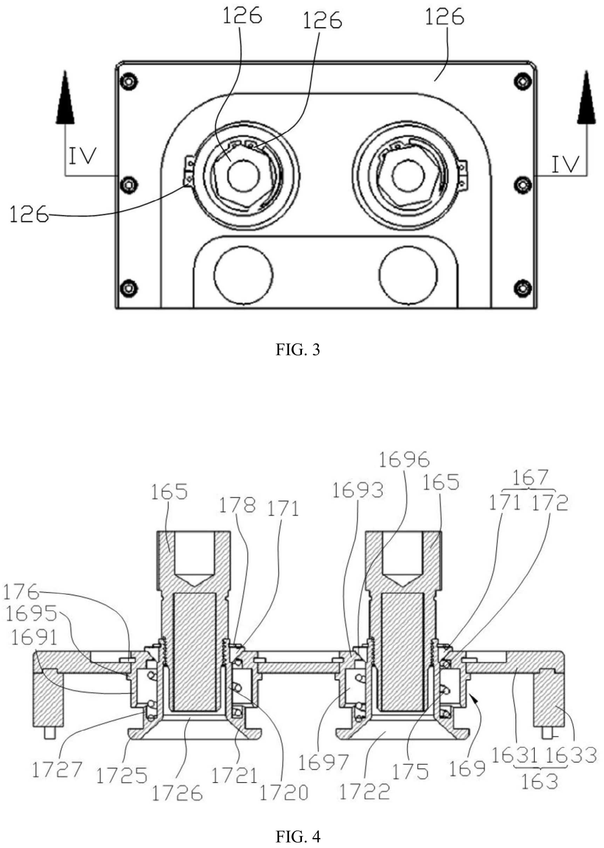

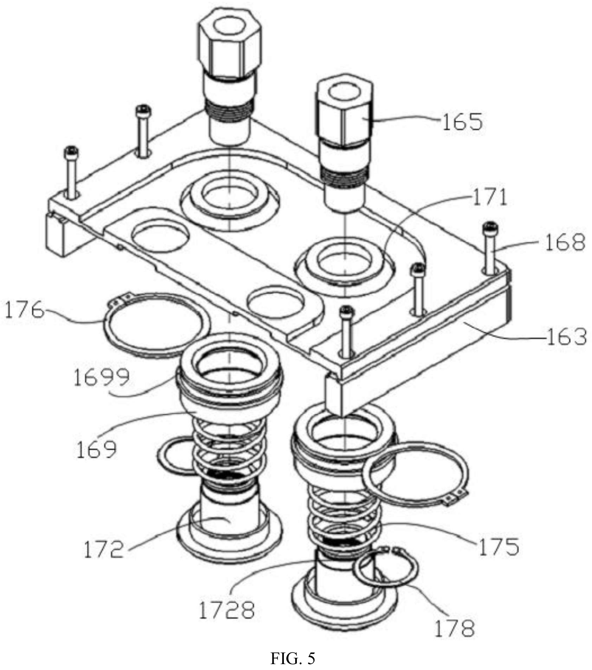

Please refer to FIGS. 3 to 5, in this embodiment, the trolley docking portion 16 includes a connector mounting plate 163, a collection-end connector 165, and a floating connector 167. The collection-end connector 165 is connected to the waste collection container. The floating connector 167 is sleeved on the collection-end connector 165 and mounted on the connector mounting plate 163. The floating connector 167 is used to guide the docking-end connector of the docking device 30 to connect with the collection-end connector 165. When the floating connector 167 is not correspondingly connected to the collection-end connector 165, the floating connector 167 is in the initial position. During the process of connecting to the docking-end connector, the floating connector 167 can float and move relative to the connector mounting plate 163, guiding the docking-end connector to align and connect with the corresponding collection-end connector 165. Specifically, in this embodiment, there are two collection-end connectors 165 and two docking-end connectors. One of the collection-end connectors 165 is used to discharge waste from the waste collection container, and the other one of the collection-end connectors 165 is used to introduce cleaning fluid for cleaning the waste collection container. The two collection-end connectors 165 are connected to the two docking-end connectors in a one-to-one correspondence. Correspondingly, there are two floating connectors 167, which are used to respectively guide the two docking-end connectors to connect with the two collection-end connectors 165.

Specifically, the connector mounting plate 163 can be fixedly connected to the trolley main frame 13 via fasteners 168.

Specifically, the trolley docking portion 16 further includes a floating connector mounting member 169. The floating connector 167 includes a floating port 171 and a floating guiding member 172. The floating connector mounting member 169 is fixed on the connector mounting plate 163. The floating connector mounting member 169 includes a positioning portion 1691 extending into the mating space 161. The floating guiding member 172 is sleeved on the collection-end connector 165 and passes through the floating connector mounting member 169 for guiding the docking-end connector of the docking device 30. The floating port 171 is fixedly connected to the floating guiding member 172. The floating port 171 is floatingly matched with a mounting hole of the floating connector mounting member 169 to engage with the floating connector mounting member 169 in the initial state. The floating connector 167 is floatingly disposed in the mounting hole formed by the floating connector mounting member 169, allowing the floating connector 167 to swing relative to the connector mounting plate 163 in any direction within a certain range, thereby achieving guidance and position correction for the docking-end connector. When the docking-end connector of the docking device 30 is not connected, the floating port 171 is engaged with the floating connector mounting member 169, and the floating connector 167 is in the initial position. During the process of connecting to the docking-end connector, the floating port 171 moves relative to the floating connector mounting member 169 for guiding the docking-end connector to align with the corresponding collection-end connector 165 until the connection is completed, thereby forming a cleaning path or a waste discharge path.

Specifically, a first elastic member 175 is provided between the floating connector mounting member 169 and the floating connector 167 to reset the floating connector 167, causing the floating port 171 to restore from the movable state to the state of engaging with the floating connector mounting member 169. Specifically, two ends of the first elastic member 175 abut against the floating connector mounting member 169 and the floating port 171, respectively. The first elastic member 175 may be a compression spring. When the docking-end connector of the docking device 30 is not connected and the floating connector 167 is in the initial position, the first elastic member 175 is in the initial state (i.e., the first elastic member 175 is not compressed or only slightly compressed). During the process of connecting to the docking-end connector, the floating connector 167 floats and the first elastic member 175 is compressed. After the docking-end connector is removed, the elastic force accumulated by the compressed first elastic member 175 restores the floating connector 167 to the initial state.

Specifically, two mounting holes may be formed on the connector mounting plate 163 for mounting two floating connector mounting members 169 respectively, thereby arranging the two floating connectors 167 correspondingly, so that the two collection-end connectors 165 are connected to the two floating connectors 167 respectively. Specifically, the connector mounting plate 163 includes a flat plate 1631 and guide plates 1633. The two guide plates 1633 are connected to both ends of the flat plate 1631 respectively and extend toward one side of the flat plate 1631. The mounting holes are formed on the flat plate 1631.

Specifically, the collection-end connector 165 and the floating connecto 167 are fixedly connected to form an integrated structure. The periperal walls of the collection-end connector 165 and the floating connector 167 may be integrally formed or connected by threads.

Specifically, the floating connector mounting member 169 includes a fixing top portion 1693, which is fixedly connected to the connector mounting plate 163. Specifically, a limiting portion 1695 is formed at the junction of the fixing top portion 1693 and the positioning portion 1691. The limiting portion 1695 abuts against one side surface of the connector mounting plate 163, and the other side surface of the connector mounting plate 163 is provided with a first clamping member 176 that abuts against the connector mounting plate 163 and clamps the fixing top portion 1693 of the floating connector mounting member 169. In this way, the fixing top portion 1693 is fixedly connected to the connector mounting plate 163 through the limiting portion 1695 and the first clamping member 176. Specifically, the first clamping member 176 may be a circlip. Specifically, a mating hole 1696 is provided in the fixing top portion 1693. The mating hole 1696 is a tapered hole with a larger top and a smaller bottom, or the inner wall of the mating hole 1696 is an inclined surface. In other words, at least part of the inner surface of the mating hole 1696 faces away from the mating space 161, and the dimension of the mating hole 1696 gradually decreases. A cavity 1697 communicating with the mating hole 1696 is formed within the positioning portion 1691. The first elastic member 175 is accommodated in the cavity 1697 with one end abutting against the fixing top portion 1693. The floating connector mounting member 169 is provided with a first clamping groove 1699 for holding the first clamping member 176.

Specifically, the floating port 171 includes a mating surface for mating with the mating hole 1696 of the floating connector mounting member 169. The mating surface is a tapered surface or an inclined surface. In other words, the mating surface faces the mating space 161 and is at least partially inclined. The mating surface of the floating port 171 can be engaged with the inner surface of the mating hole 1696, or can be disengaged from the mating hole 1696 as the floating port 171 floats.

Specifically, the floating guiding member 172 includes a sleeve portion 1720 and a guiding end 1721 for guiding the docking-end connector. One end of the sleeve portion 1720 is connected to the collection-end connector 165, and the guiding end 1721 is connected to the other end of the sleeve portion 1720. The guiding end 1721 is provided with a guiding opening 1722. The guiding opening 1722 may be a conical hole, or the inner wall of the guiding opening 1722 is composed of multiple inclined surfaces, i.e., the guiding opening 1722 has a bell-mouth structure, and the bell-mouth can be either a curved surface or a tapered surface. In this way, the guiding opening 1722 can guide the docking-end connector of the docking device 30 during docking, facilitating position alignment in the docking process. One end of the floating guiding member 172 protrudes from one end of the floating connector mounting member 169 into the mating space 161. The floating guiding member 172 further includes a limiting edge 1725 that matches with the positioning portion 1691 to limit the upward floating space of the floating connector 167 during floating. The limiting edge 1725 is provided on the outer wall of the guiding end 1721. In this embodiment, the outer diameter of the limiting edge 1725 is larger than the outer diameter of the positioning portion 1691. When the floating guiding member 172 moves upward to abut against the positioning portion 1691, the positioning portion 1691 can block the floating guiding member 172 from moving further upward. Specifically, a chamfered portion 1726 is formed at one end of the guiding end 1721 away from the limiting edge 1725. Specifically, the diameter of the guiding opening 1722 at its smallest position ranges from 40 mm to 60 mm. Such a relatively large size of the guiding opening 1722 provides a larger fault tolerance margin. In addition, the diameter of the floating connector 167 ranges from 30 mm to 38 mm, and the inner diameter of the positioning portion 1691 ranges from 50 mm to 70 mm. This allows the floating connector 167 to have a relatively large tilt angle range before completing docking, making it easier to guide the floating connector 167 to be connected.

Specifically, the floating guiding member 172 further includes an outer tube 1727, which is connected to the outer wall of the guiding end 1721. The outer tube 1727 is sleeved outside the guiding end 1721 with a gap formed between the outer tube 1727 and the guiding end 1721. One end of the first elastic member 175 abuts against the gap between the outer tube 1727 and the guiding end 1721.

Specifically, the surface of one side of the floating port 171 is provided with a second clamping member 178 that abuts against the floating port 171 and clamps the floating guiding member 172. In this way, the floating port 171 is fixedly connected to the floating guiding member 172 through the second clamping member 178. The second clamping member 178 may be a circlip. The floating guiding member 172 is provided with a second clamping groove 1728 for holding the second clamping member 178.

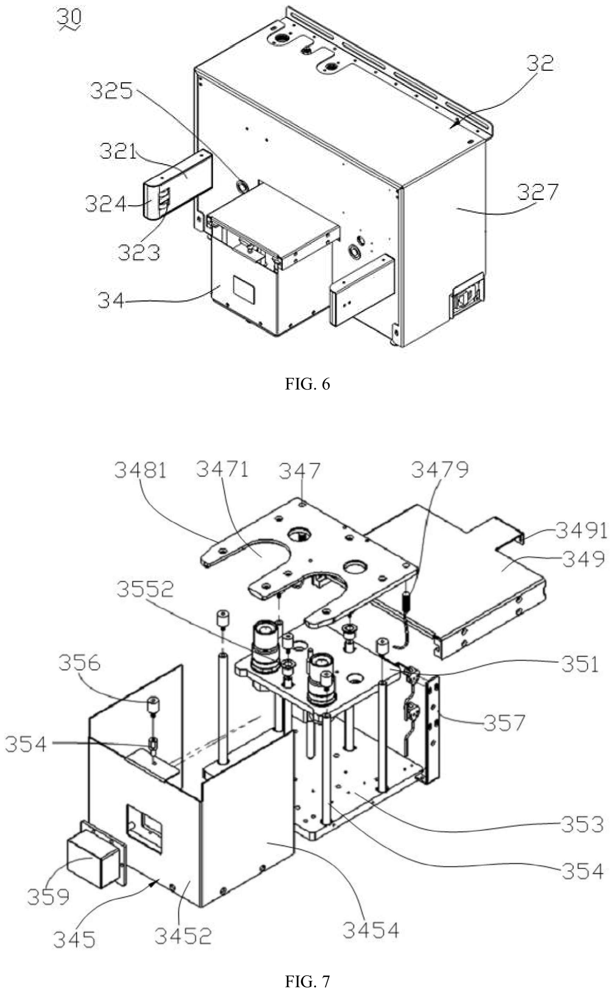

Please refer to FIGS. 6 to 8, in this embodiment, the docking device 30 includes a liquid discharge station main body 32 and a mating head 34, with the mating head 34 disposed on one side of the liquid discharge station main body 32. The mating head 34 is configured to match with the trolley docking portion 16 of the waste collection apparatus 10. Specifically, the mating head 34 is inserted into the mating space 161 of the trolley docking portion 16 to achieve preliminary alignment between the docking device 30 and the waste collection apparatus 10.

In this embodiment, the liquid discharge station main body 32 is further provided with liquid discharge station guiding portions 321. Two liquid discharge station guiding portions 321 are respectively disposed on both sides of the liquid discharge station main body 32, so as to match with both sides of the trolley housing 12 respectively, thereby guiding the docking device 30 during the process in which the mating head 34 of the docking device 30 enters the mating space 161. Specifically, the side surfaces of the two liquid discharge station guiding portions 321 facing each other are further provided with a second anti-collision portion 323. The second anti-collision portion 323 may specifically be anti-collision rollers, so as to prevent collision damage to the trolley housing 12 during docking. Specifically, a chamfered portion 324 is formed at the end of the liquid discharge station guiding portion 321 that is away from the liquid discharge station main body 32 to achieve better guiding performance. By providing the first anti-collision portions and the second anti-collision portions, the mating head can be prevented from colliding with the inner sidewall of the trolley docking portion of the waste collection apparatus when the mating head of the docking device enters the mating space, and meanwhile, the mating head is prevented from colliding with the trolley housing of the waste collection apparatus, thus avoiding mutual collision between the waste collection apparatus and the mating head.

Specifically, the liquid discharge station main body 32 is further provided with a magnetic member 325. Specifically, the magnetic member 325 is disposed on one side of the mating head 34. When the docking device 30 comes into contact with the waste collection apparatus 10, the magnetic member 325 is attracted to the magnetic stopper 126 of the waste collection apparatus 10, and at this time, the mating head 34 has fully entered the mating space 161.

Specifically, the liquid discharge station main body 32 includes a liquid discharge station housing 327 and a liquid discharge device disposed inside the liquid discharge station housing 327.

In this embodiment, the mating head 34 includes a docking-end connector 341 and an actuator 343. The docking-end connector 341 is connected to the actuator 343. The actuator 343 is disposed inside the mating head 34 and capable of free movement in specific directions, thereby driving the docking-end connector 341 to move for docking with the collection-end connector 165. After the mating head 34 is aligned with the trolley docking portion 16, the actuator 343 is activated to bring the docking-end connector 341 into contact with the floating connector 167, and the docking-end connector 34 then drives the floating connector 167 to float and move, thereby guiding the docking-end connector 341 to connect with the collection-end connector 165.

In this embodiment, one of the docking-end connectors 341 docks with one collection-end connector 165 to form a waste discharge path for transporting the waste in the waste collection container, and the other one of the docking-end connectors 341 docks with the other collection-end connector 165 to form a cleaning path for cleaning the waste collection container and transporting the cleaning fluid for the waste collection container. Specifically, one of the docking-end connectors 341 is connected to a cleaning interface on the liquid discharge station main body 32 for injecting cleaning fluid. The docking device 30 further includes a suction pump (not shown), which is connected to one of the docking-end connectors 341 and allows the waste sucked by this docking-end connector 341 to be discharged through a discharge connector on the liquid discharge station main body 32.

Specifically, the actuator 343 can be a pneumatic pump, a stepping motor, or other components or mechanical structures capable of telescopic movement. A pneumatic pump is preferred in this embodiment, as it can reduce the number of circuit components of the docking device 30, thereby decreasing circuit faults and the potential risks posed by complex circuits to the device. In addition, the speed and force of the pneumatic pump during propulsion are more conducive to the guiding effect of the floating connectors 167 on the docking-end connectors 341.

Specifically, the end of the docking-end connector 341 that docks with the collection-end connector 165 is provided with a chamfered portion, which serves as a guide fuction during the docking process between the docking-end connector 341 and the floating connector 167 to facilitate docking.

In this embodiment, the mating head 34 further includes a mating head housing 345, and both the docking-end connectors 341 and the actuator 343 are disposed inside the mating head housing 345. The mating head housing 345 includes a front panel 3452 and side panels 3454 connected to both sides of the front panel 3452. The rear end of the mating head housing 345, opposite to the front panel 3452, is connected to the liquid discharge station main body 32.

In this embodiment, the mating head 34 further includes an adapter plate 347, which is provided with two guiding grooves 3471, and the docking-end connectors 341 can pass through the guiding grooves 3471 correspondingly. Specifically, the two guiding grooves 3471 are used for the two docking-end connectors 341 to pass through, respectively. Preferably, the guiding groove 3471 is approximately U-shaped, with an arc-shaped inner end and a wedge-shaped front end 3473, such that the opening width of the guiding groove gradually decreases from the front end to the inner end. This structure allows the floating connector 167 to be gradually aligned with the guiding groove 3471 without collision when the mating head 34 is pushed into the mating space 161 of the trolley docking portion 16.

Specifically, the adapter plate 347 is provided with an elastic member hanging hole 3477.

Specifically, the adapter plate 347 is further provided with a first magnetic switch 3479 and a second magnetic switch 3480, both of which are disposed at the rear end of the adapter plate 347 (i.e., the end away from the guiding grooves 3471).

In this embodiment, the mating head 34 further includes a cover plate 349, which is movably disposed above the adapter plate 347. When the mating head 34 is pushed into the mating space 161, the waste collection apparatus 10 pushes the cover plate 349, displacing the cover plate 349 from the adapter plate 347. After the mating head 34 is separated from the mating space 161, the cover plate 349 can reset to a position covering the adapter plate 349 and the docking-end connectors 341.

Specifically, the cover plate 349 is provided with sliding grooves, and guide rails 3481 slidably fitted in the sliding grooves are provided on both sides of the adapter plate 347 to guide the movement of the cover plate 349.

Specifically, a second elastic member (not shown) is provided between the adapter plate 347 and the cover plate 349. The second elastic member is used to provide elastic force for the automatic resetting of the cover plate 349 after the mating head 34 is separated from the mating space 161. More specifically, during the process of pushing the mating head 34 into the mating space 161, the elastic member is gradually stretched, and after the mating head 34 is separated from the mating space 161, the elastic member retracts to release the elastic force. Specifically, the cover plate 349 is provided with an elastic member hanging portion 3491, and the two ends of the second elastic member are respectively connected to the elastic member hanging portion 3491 and the elastic member hanging hole 3477 on the adapter plate 347.

Specifically, the cover plate 349 is further provided with a first magnetic element 3493 and a second magnetic element 3494. The first magnetic element 3493 is disposed at the rear end of the cover plate 349 (i.e., the end of the cover plate 349 facing away from the trolley docking portion 16), and the second magnetic element 3494 is disposed at the front end of the cover plate 349 (i.e., the end of the cover plate 349 facing the trolley docking portion 16). When the first magnetic switch 3479 senses the first magnetic element 3493, it indicates that the docking device 30 and the waste collection apparatus 10 are ready for docking; when the second magnetic switch 3480 senses the second magnetic element 3494, it indicates that the mating head 34 has fully entered the mating space 161, and the actuator 343 can start operating for docking.

In this embodiment, the mating head 34 further includes a moving plate 351. The docking-end connectors 341 are disposed on the moving plate 351, and the actuator 343 drives the moving plate 351 to move up and down, thereby driving the docking-end connectors 341 to move for docking with the collection-end connectors 165.

In this embodiment, the mating head 34 further includes a bottom plate 353, support columns 354, and guiding columns 355. The moving plate 351 is located between the bottom plate 353 and the adapter plate 347. Both the support columns 354 and the guiding columns 355 are fixed relative to the bottom plate 353. The adapter plate 347 is fixedly connected to the support columns 354, and the moving plate 351 is movably inserted through the guiding columns 355. Specifically, one end of each guiding column 355 is also connected to the adapter plate 347. A buffer pad 356 is provided at the end of each support column 354 that is connected to the adapter plate 347 for reducing vibration and muffle noise when the moving plate 351 rises to a position close to the adapter plate 347. Specifically, one end of the support column 354 located between the two guiding grooves 3471 is connected to the mating head housing 345, and one end of each of the remaining support columns 354 is connected to the bottom plate 353.

Specifically, a bushing 3552 is sleeved on each guiding column 355 to reduce friction between the moving plate 351 and the guiding column 355 during the up-and-down movement of the moving plate 351.

In this embodiment, the mating head 34 further includes a sensor bracket 357, on which a first sensor 3571 and a second sensor 3573 are arranged at intervals. A sensor stopper 3511 is provided on the moving plate 351. The first sensor 3571 and the second sensor 3573 are configured to sense the sensor stopper 3511. When the first sensor 3571 detects the sensor stopper 3511, it indicates that the collection-end connector 165 and the docking-end connector 341 have been successfully docked. When the operation of the liquid discharge station main body 32 is completed, the actuator 343 drives the moving plate 351 and the docking-end connector 341 to move downward. The sensor stopper 3511 moves away from the first sensor 3571 until the second sensor 3573 detects the sensor stopper 3511, which means the moving plate 351 and the docking-end connector 341 have returned to their initial positions. The first sensor 3571 and the second sensor 3573 can be photoelectric sensors. When the first sensor 3571 detects the sensor stopper 3511, the first sensor 3571 lights up; when the second sensor 3573 detects the sensor stopper 3511, the second sensor 3573 lights up. It should be understood that the sensor bracket 357 may also be omitted, and the first sensor 3571 and the second sensor 3573 may be arranged on other components (e.g., the mating head housing 345), provided that the first sensor 3571 and the second sensor 3573 are relatively fixed with respect to the adapter plate 347.

In this embodiment, the mating head 34 further includes a charging port 359 configured to charge the waste collection apparatus 10 via the charging receiving portion 162 of the trolley docking portion 16. Specifically, the charging port 359 can be a wireless charging port, which only needs to be in contact with or close to the charging receiving portion 162 to perform charging.

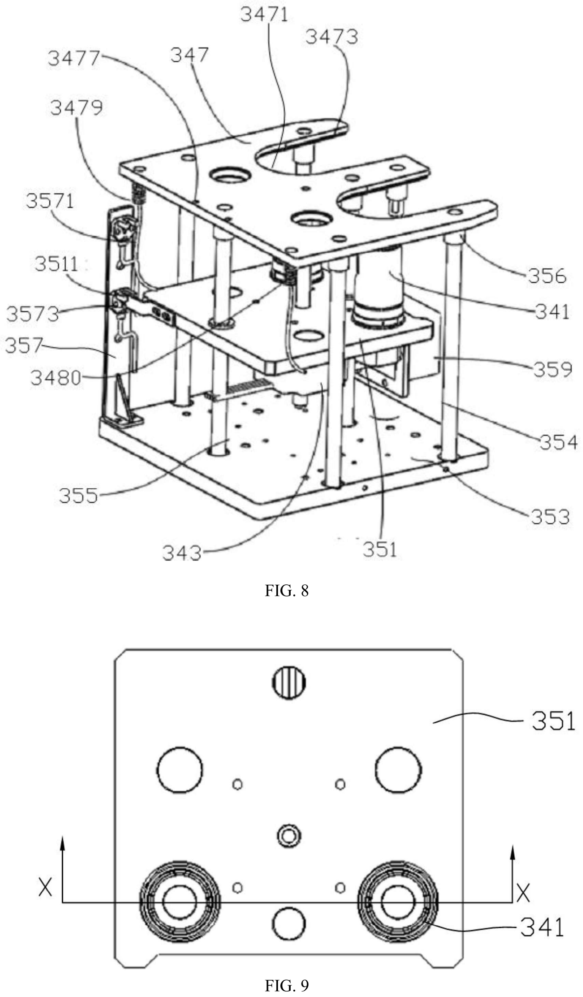

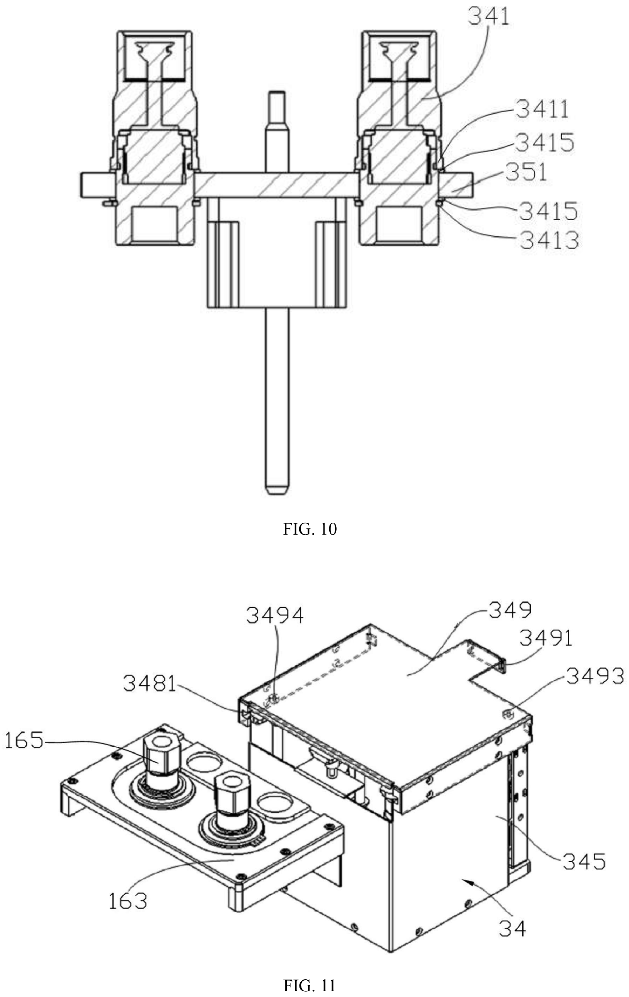

In this embodiment, please refer to FIGS. 9 and 10, a step portion 3411 is provided on the outer wall of the docking-end connector 341. One end of the docking-end connector 341 passes through the moving plate 351, with the step portion 3411 abutting against the surface of one side of the moving plate 351. A fastener 3413 is clamped to the docking-end connector 341 and abuts agains the surface of the other side of the moving plate 351, thereby fixing the docking-end connector 341 on the moving plate 351. Specifically, a gasket 3415 is further provided between the step portion 3411 and the moving plate 351, as well as between the fastener 3413 and the moving plate 351. The fastener 3413 can be a circlip, and the gasket 3415 can be a tetrafluoroethylene gasket.

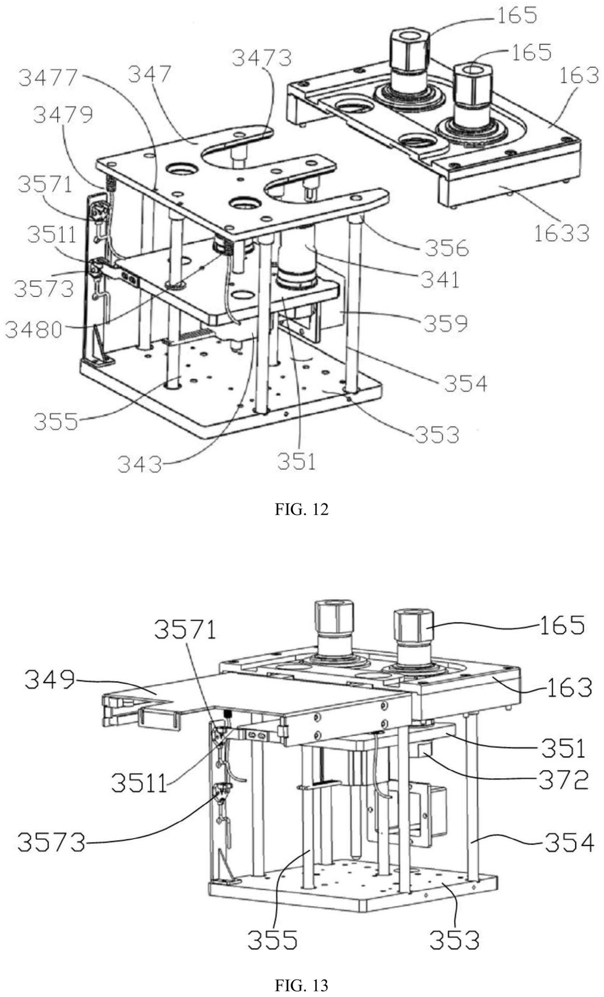

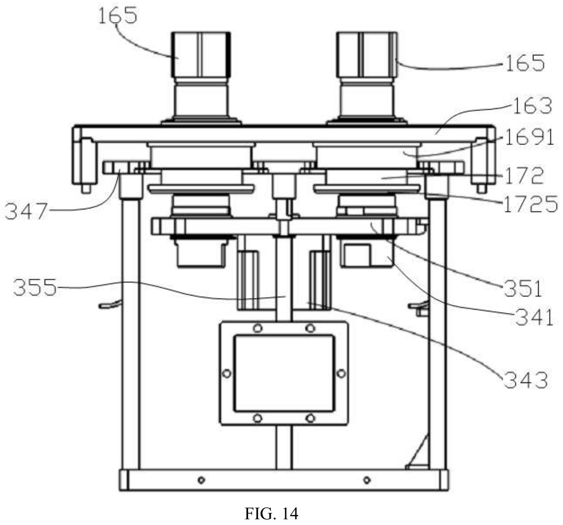

In this embodiment, please refer to FIGS. 11 and 12, when the mating head 34 has not yet entered the mating space 161 of the trolley docking portion 16, the floating connector mounting member 169 is located on one side of the mating head 34, and the cover plate 349 covers the adapter plate 347. Then, the mating head 34 is gradually inserted into the mating space 161 of the trolley docking portion 16. After the connector mounting plate 163 comes into contact with the cover plate 349, as the mating head 34 moves further into the mating space 161, the connector mounting plate 163 gradually pushes the cover plate 349 to move relative to the adapter plate 347. During this process, the floating connector 167 gradually enters the guiding groove 3471 until the collection-end connector 165 is roughly aligned with the docking-end connector 341. The guiding groove 3471 plays a guiding role for the floating connector 167 to enter, and at this time, the positioning portion 1691 of the floating connector mounting member 169 matches with the guiding groove 3471 of the adapter plate 347 to achieve preliminary positioning. Next, please refer to FIGS. 13 and 14, the actuator 343 is then activated. The actuator 343 operates to bring the docking-end connector 341 into contact with the floating connector 167, and causes the floating connector 167 to float, thereby guiding the docking-end connector 341 to connect with the collection-end connector 165.

In this embodiment, since the positioning portion 1691 is provided on the floating connector mounting member 169, after the positioning portion 1691 is aligned with the guiding groove 3471, the floating space of the floating connector 167 is not constrained by the adapter plate 347 or the guiding groove 3471, thereby not limiting the floating space of the floating connector 167, to ensure a large fault tolerance margin during docking and prevent the floating connector 167 from jamming during the docking process. Before the docking is completed, the horizontal floating space of the floating connector 167 is related to the distance between the inner surface of the floating connector mounting member 169 and the outer surface of the floating guiding member 172; the vertical floating space of the floating connector 167 is related to the distance between the lowermost surface of the floating connector mounting member 169 and the limiting edge 1725 of the floating guiding member 172. The larger these two distances are, the larger the floating space will be. The specific distances can be set according to the actual dimensions of the device and the average conditions of the intended application scenarios. The floating space can be completely free from the restriction of the mating head 34 of the liquid discharge station, and a very wide floating space can be achieved by increasing these two distances if necessary.

It should be understood that the structures or structural features involved in the above embodiments can be arbitrarily superimposed without conflict.

In this description, unless otherwise explicitly specified and limited, the terms "mount" "connect" shall be interpreted in a broad sense. For example, they may refer to fixed connection, detachable connection, or integral connection; mechanical connection or electrical connection; direct connection or indirect connection via an intermediate medium; or internal connection between two components. Those of ordinary skill in the art can understand the specific meanings of the above terms according to specific situations.

In this description, the terms "upper", "lower", "front", "rear", "left", "right", "top", "bottom", "inner", "outer", "vertical" and "horizontal" indicate orientations or positional relationships based on those shown in the accompanying drawings, which are only for the convenience of clarifying and describing the technical solutions, and thus should not be construed as limitations to this invention.

In this description, the terms "comprise", "include" or any other variants thereof are intended to cover non-exclusive inclusion, such that a structure, assembly or device that includes a list of elements not only includes those elements but also includes other elements not explicitly listed, or elements inherent to such structure, assembly or device.

The above are only specific embodiments of this invention, but the protection scope of this invention is not limited thereto. Any person skilled in the art can easily think of changes or substitutions within the technical scope disclosed by this invention, which shall fall within the protection scope of this invention. Therefore, the protection scope of this invention shall be subject to the protection scope defined by the claims.

## Claims

1. A waste collection apparatus for collecting waste generated during medical procedures and for docking with a docking device (30), wherein the waste collection apparatus comprises a trolley docking portion (16), the trolley docking portion (16) forms a mating space (161) for accommodating a mating head (34) of the docking device (30); the trolley docking portion (16) comprises a connector mounting plate (163), a collection-end connector (165), a floating connector (167), and a floating connector mounting member (169), wherein the floating connector mounting member (169) is fixed on the connector mounting plate (163), the collection-end connector (165) is connected to the floating connector (167), the floating connector (167) is floatingly movably mounted on the floating connector mounting member (169), and the floating connector (167) is configured to guide a docking-end connector (341) of the docking device (30) to connect with the collection-end connector (165), wherein the floating connector mounting member (169) comprises a positioning portion (1691) extending into the mating space (161).

2. The waste collection apparatus according to claim **1,** wherein the floating connector (167) is floatingly disposed in a mounting hole formed by the floating connector mounting member (169), the floating connector (167) comprises a floating port (171) and a floating guiding member (172), the floating port (171) is fixedly connected to the floating guiding member (172), the floating guiding member (172) is sleeved on the collection-end connector (165) and passes through the floating connector mounting member (169) for guiding the docking-end connector of the docking device (30); and the floating port (171) is floatingly matched with the mounting hole of the floating connector mounting member (169) to engage with the floating connector mounting member (169) or move relative to the floating connector mounting member (169).

3. The waste collection apparatus according to claim **2,** wherein a first elastic member (175) is provided between the floating connector mounting member (169) and the floating connector (167), and the first elastic member (175) is configured to provide an elastic force for resetting the floating connector (167) so as to restore the floating port (171) from a movable state to a state of engaging with the floating connector mounting member (169).

4. The waste collection apparatus according to claim **2,** wherein the floating connector mounting member (169) comprises a fixing top portion (1693), the fixing top portion (1693) is fixedly connected to the connector mounting plate (163), a mating hole (1696) is provided in the fixing top portion (1693), at least part of an inner surface of the mating hole (1696) faces away from the mating space (161), and a dimension of the mating hole (1696) gradually decreases, the floating port (171) comprises a mating surface for mating with the mating hole (1696), the mating surface faces the mating space (161) and is at least partially inclined; and the mating surface of the floating port (171) engages with the inner surface of the mating hole (1696), or disengages from the mating hole (1696) as the floating port (171) floats.

5. The waste collection apparatus according to claim **2,** wherein the floating guiding member (172) comprises a sleeve portion (1720) and a guiding end (1721) for guiding the docking-end connector (341), one end of the sleeve portion (1720) is connected to the collection-end connector (165), the guiding end (1721) is connected to the other end of the sleeve portion (1720), the guiding end (1721) is provided with a guiding opening (1722), and the guiding opening (1722) has a bell-mouth structure.

6. The waste collection apparatus according to claim **5,** wherein a diameter of the guiding opening (1722) at its smallest position ranges from 40 mm to 60 mm.

7. The waste collection apparatus according to claim **6,** wherein a diameter of the floating connector (167) ranges from 30 mm to 38 mm, and an inner diameter of the positioning portion (1691) ranges from 50 mm to 70 mm.

8. The waste collection apparatus according to claim **5,** wherein one end of the floating guiding member (172) protrudes from one end of the floating connector mounting member (169) into the mating space (161), the floating guiding member (172) further comprises a limiting edge (1725) that matches with the positioning portion (1691) to limit an upward floating space of the floating connector (167) during floating, and the limiting edge (1725) is provided on an outer wall of the guiding end (1721).

9. The waste collection apparatus according to claim **8,** wherein a chamfered portion (1726) is formed at one end of the guiding end (1721) away from the limiting edge (1725).

10. The waste collection apparatus according to claim **1,** wherein the waste collection apparatus further comprises a trolley housing (12), a waste collection container disposed inside the trolley housing (12), a trolley main frame (13), and a travel mechanism (14) arranged at the bottom of the trolley main frame (13), wherein the trolley docking portion (16) is mounted on the trolley main frame (13).

11. A waste liquid collection and treatment system comprising the waste collection apparatus (10) according to any one of claims **1** to **10** and a docking device (30), wherein the docking device (30) comprises a mating head (34), the mating head (34) is configured to be inserted into the mating space (161) of the trolley docking portion (16), the mating head (34) comprises a docking-end connector (341) for engaging with the collection-end connector (165) and an adapter plate (347), a guiding groove (3471) allowing the docking-end connector (341) to pass through is provided in the adapter plate (347), and the positioning portion (1691) of the floating connector mounting member (169) matches with the guiding groove (3471) of the adapter plate (347).

12. The waste liquid collection and treatment system according to claim **11,** wherein the guiding groove (3471) is U-shaped, with a wedge-shaped front end (3473), and the guiding groove (3471) has an opening width gradually decreasing from the front end to an inner end thereof.

13. The waste liquid collection and treatment system according to claim **11,** wherein the mating head (34) further comprises an actuator (343), which is connected to the docking-end connector (341) and configured to drive the docking-end connector (341) to move for docking with the collection-end connector (165).

14. The waste liquid collection and treatment system according to claim **13,** wherein the mating head (34) further comprises a moving plate (351), the docking-end connector (341) is disposed on the moving plate (351), and the actuator (343) drives the moving plate (351) to move up and down.

15. The waste liquid collection and treatment system according to claim **14,** wherein the mating head (34) further comprises a bottom plate (353), support columns (354), and guiding columns (355), wherein the moving plate (351) is located between the bottom plate (353) and the adapter plate (347), the support columns (354) and the guiding columns (355) are fixed relative to the bottom plate (353), the adapter plate (347) is fixedly connected to the support columns (354), and the moving plate (351) is movably inserted through the guiding columns (355).

16. The waste liquid collection and treatment system according to claim **15,** wherein a buffer pad (356) is provided at one end of each of the support columns (354) and the guiding columns (355) that is connected to the adapter plate (347).

17. The waste liquid collection and treatment system according to claim **14,** wherein the mating head (34) further comprises a first sensor (3571) and a second sensor (3573), which are relatively fixed to the adapter plate (347), the first sensor (3571) and the second sensor (3573) are arranged at intervals, a sensor stopper (3511) is provided on the moving plate (351), the first sensor (3571) and the second sensor (3573) are configured to sense the sensor stopper (3511); when the first sensor (3571) detects the sensor stopper (3511), the collection-end connector (165) and the docking-end connector (341) have been successfully docked; and when the second sensor (3573) detects the sensor stopper (3511), the moving plate (351) and the docking-end connector (341) have returned to their initial positions.

18. The waste liquid collection and treatment system according to claim **11,** wherein the mating head (34) further comprises a cover plate (349), the cover plate (349) is movably disposed above the adapter plate (347), a second elastic member is provided between the adapter plate (347) and the cover plate (349), and the second elastic member is configured to provide an elastic force for automatically resetting the cover plate (349) to a position covering the adapter plate (347).

19. The waste liquid collection and treatment system according to claim **18,** wherein the cover plate (349) is provided with a first magnetic element (3493) and a second magnetic element (3494), the first magnetic element (3493) is disposed at a rear end of the cover plate (349), the second magnetic element (3494) is disposed at a front end of the cover plate (349); the adapter plate (347) is provided with a first magnetic switch (3479) for sensing the first magnetic element (3493) when the cover plate (349) covers the adapter plate (347) and a second magnetic switch (3480) for sensing the second magnetic element (3494) when the cover plate (349) moves to a position displaced from the adapter plate (347), and the first magnetic switch (3479) and the second magnetic switch (3480) are both disposed at the rear end of the adapter plate (347).

20. The waste liquid collection and treatment system according to claim **11,** wherein first anti-collision portions (127) are disposed on both sides of the mating space (161); the docking device (30) comprises a liquid discharge station main body (32), the mating head (34) is connected to one side of the liquid discharge station main body (32), the liquid discharge station main body (32) is provided with two second anti-collision portions (323) located on both sides of the mating head (34), and a distance between the two second anti-collision portions (323) corresponding to a width of the trolley housing (12).

21. The waste collection and treatment system according to claim **20,** wherein the liquid discharge station main body (32) is provided with two liquid discharge station guiding portions (321) respectively disposed on both sides of the mating head (34), and the two second anti-collision portions (323) are provided on side surfaces of the two liquid discharge station guiding portions (321) facing each other.

22. The waste collection and treatment system according to claim **21,** wherein a chamfered portion (324) is formed at one end of the liquid discharge station guiding portion (321) away from the liquid discharge station main body (32).

23. The waste collection and treatment system according to claim **20,** wherein the first anti-collision portions (127) and the second anti-collision portions (323) are rollers.
